# EUROPEAN PATENT APPLICATION

(11) **EP 4 011 291 A1**
(43) Date of publication of application: **15.06.2022**
(21) Application number: 20212561.3
(22) Date of filing: 08.12.2020
(51) Int. Cl.: A61B 5/11

(54) **SYSTEM AND METHOD FOR MONITORING BODY POSTURE AND MOVEMENT**

(71) Applicant: Alcass Health Solutions Limited, Cork (IE)
(72) Inventor: Allen, Breffni, Cork, (IE); Harnett, Edward, Cork, (IE)
(74) Representative: Purdylucey Intellectual Property

(57) **Abstract**

The present invention relates to a system and method for monitoring, measuring, and analysing the sitting posture of a user and the number of steps taken by the user while in motion. The system as per the present invention comprises a display means, a plurality of data sensors, a processing means and a memory means and the number of minutes spent sitting in same position before prompting the user to get up and move by setting a predetermined number of steps. The data sensors are configured to, continuously monitor the seating posture of a user by measuring a plurality of parameters in real time, measure the time for which the user remains seated, and the number of steps taken by the user. Based on the inputs from the data sensors, the processing means triggers feedback to the users in case of a poor sitting posture or if the user has been in sitting posture beyond a threshold time period. The system as per the present invention further provides personalized wellness suggestions based on historic values of measured parameters and behavioural patterns.

## Description

### Field

The present disclosure relates to a system and method for monitoring body posture and movement, and more particularly to a system and method for monitoring, measuring, and analysing the sitting posture of a user.

### Background

Sitting for long periods of time has been branded the "new smoking" due to its purported health risks. Health concerns that may arise due to sedentary lifestyles are well researched and well known. Such health concerns include obesity and conditions such as increased blood pressure, high blood sugar, excess visceral fat, and abnormal cholesterol levels. Prolonged periods of sitting have also been linked to increased risk of death due to cardiovascular disease and cancer.

An optimum sitting posture is also known to be equally important to avoid undue stress on muscles, joints, and nerves. Poor sitting posture or slouching has often been connected to persistent back pain among working adults. It is estimated that about nine out of ten adults experience back pain at some point in their life, and that five out of ten working adults have back pain every year. A poor sitting posture can also lead to tension and pain in neck, upper back, lower back, and shoulders, and could lead to constricted nerves and a curvature of the spine in the long term.

Wearable devices which measure the postural angles of a user are known in the art. Wearable devices which measure the number of steps taken by a user are also known in the art, for example pedometers. However, none of these known devices measure the postural angles of the user, or measures time sitting to provide feedback to the user in case of slouching, measure steps taken by a user, and provide qualitative wellness insights to the user.

It is an object of the invention to provide a system and method to overcome at least one of the above mentioned problems.

### Summary

In a preferred embodiment of the present invention, a system for monitoring body posture and a lack of movement is provided, as set out in the appended claims.

The system comprises a display means, a processing means, a plurality of data sensors, and a memory means. The display means is operatively coupled to the processing means and is configured to display a plurality of notifications generated by the processing means. The display means also enables a user to predefine threshold values for a plurality of parameters indicating an ideal sitting posture of the user. The predefined threshold values correspond to postural angles when the user is seated in an optimal posture. The plurality of data sensors is operatively coupled to the processing means and is configured to continuously monitor the sitting posture of the user by measuring the plurality of parameters in real time. The data sensors are further configured to measure the time for which the user is in sitting position and to measure the number of steps taken by the user while in motion.

The invention provides a system and method that enables a user with a discrete, non-intrusive, real time motivation, real time data useful for clinical decision making.

In an embodiment of the present invention, the plurality of parameters comprises pitch, roll, and yaw, and the plurality of data sensors comprises an accelerometer, a pressure sensor, a gyro-meter, and a magnetometer.

The memory means is operatively coupled to the display means and the processing means, and has a plurality of instructions stored on it which, based on the inputs from the data sensors, enables the processing means to triggers a feedback to the user. The feedback is triggered if the difference in real time values of the plurality of parameters and the predefined threshold values, exceeds a predefined tolerance limit. The feedback is triggered after a predetermined time interval, for example 5 seconds. The processing means is further configured to trigger a notification to the user with one or more goals to be obtained, for example to move a predetermined number of steps, if the time measured by the plurality of data sensors exceeds a predefined threshold, and to continuously analyse the parameter values and time measured by the plurality of data sensors and provide a plurality of personalized wellness advisories to the user. The memory means is further configured to store the historical values of the plurality of parameters and the historical values of time measured by the plurality of sensors.

In an embodiment of the present invention, the historic data stored in the memory means is used to train artificial intelligence and machine learning models which are in turn used to determine health risk prevention strategies for users.

In an embodiment of the present invention, the plurality of data sensors is positioned under the collar bone of the user and feedback is provided to the user through vibrations generated by a vibration motor.

In an embodiment of the present invention, a method for monitoring body posture and body movement is provided. The method comprises the steps of firstly predefining threshold values for a plurality of parameters indicating an ideal sitting posture of the user. Once the threshold values are defined, the sitting posture of the user is continuously monitored by measuring the plurality of parameters in real time using a plurality of data sensors. A feedback is triggered to the user if difference in values of the plurality of parameters measured in real time and the corresponding threshold values of the parameters, exceeds a predefined tolerance limit. The time for which the user is in sitting posture is then measured and a notification is triggered to the user with one or more goals to be obtained, for example to take a predetermined number of steps if the measured time exceeds a predefined threshold. Further, the number of steps taken by the user is measured. The real time values of the plurality of parameters, the time for which the user remains in sitting posture, and the number of steps taken by the user, are analysed and a plurality of personalized wellness advisories are provided to the user.

The feedback triggered by the present invention enables a user to maintain an optimal posture, specific to their anatomical attributes. The plurality of personalized wellness advisories prescribed to the user includes for example, exercises specific to the user based on their activity levels and posture habits, to reverse the effects of sitting and slouching all day. The present invention hence helps to prevent lifestyle related illnesses and health risks. The data analysis and statistical insights generated by the present invention can be used by employers to improve workplace wellness and ergonomics, which enhances employee productivity and lessens healthcare costs. The present invention can also be used by insurance companies to customize insurance premiums.

The present invention hence provides a robust and practical solution to problems identified in the art.

There is also provided a computer program comprising program instructions for causing a computer program to carry out the above method which may be embodied on a record medium, carrier signal or read-only memory.

### Brief Description of Drawings

The invention will be more clearly understood from the following description of an embodiment thereof, given by way of example only, with reference to the accompanying drawings, in which:-
Figure 1 is a schematic diagram of a system as per a preferred embodiment of the present invention.
Figure 2 illustrates a display means as per a preferred embodiment of the present invention.
Figure 3 illustrates a display means as per a preferred embodiment of the present invention.
Figure 4 is a flow diagram illustrating a method as per a preferred embodiment of the present invention.

### Detailed description of drawings

The present invention relates to a system and method for monitoring body posture and movement, and more particularly to a system and method for monitoring, measuring, and analysing the sitting posture of a user and the number of steps taken by the user while in motion.

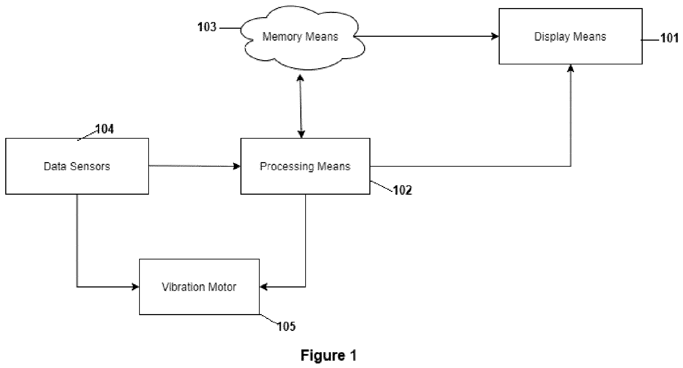

Figure 1 illustrates a system as per a preferred embodiment of the present invention. The system comprises a display means 101 operatively coupled to a processing means 102 and a memory means 103. The system is firstly calibrated by predefining threshold values for a plurality of parameters based on postural angles which indicate an ideal sitting posture of the user. The display means 101 is configured to enable a user to predefine threshold values for the parameters and is further configured to display a plurality of notifications generated by the processing means 102. The threshold values are predefined based on the axial position and orientation of a plurality of data sensors 104 which is displayed to the user through the display means 101. The plurality of parameters includes pitch, roll, and yaw.

The plurality of data sensors 104 is operatively coupled to the processing means 102 and is configured to continuously monitor the sitting posture of the user by measuring the plurality of parameters in real time. The plurality of data sensors 104 is further configured to continuously measure the time for which the user is in sitting posture and to measure the number of steps taken by the user while in motion. In an embodiment of the present invention, the plurality of data sensors 104 is positioned under the collar bone of the user. The plurality of data sensors 104 includes for example, an accelerometer, a pressure sensor, a gyro-meter, and a magnetometer. The measurements made by the plurality of sensors 104 are inputted to the processing means 102.

The processing means 102 has the non-transitory memory means 103 operatively coupled to it. The processing means 102 may be for example, a personal computer, a portable device such as a tablet computer, a laptop, a smart phone, a connected household device or any operating system based portable device or any cloud hosted enterprise business rule engine. The operating system deployed on the processing means 102 may be Windows, OSX, Linux, iOS, Android, or the like. The memory means 103 may be any internal or external device or web-based data storage mechanism adapted to store data.

The memory means 103 has a plurality of instructions stored in it which configures the processing means 102 to continuously compute the difference in real time values of the plurality of parameters and their corresponding predefined threshold values. For example, in an embodiment of the present invention, said difference is computed by the processing means 102 five times per second. If the difference in values exceeds a predefined tolerance limit, a feedback is triggered to the user after a predetermined time period, since a substantial deviation from the threshold would indicate that the user's sitting posture is poor or in other words the user is slouching. The feedback is triggered after a time delay, for example five to 20 seconds, to avoid feedback in cases wherein the user has inadvertently changed his sitting posture and immediately returns to the optimum posture. In an embodiment of the present invention, the time delay, or the predetermined time period for triggering feedback to the user is five (5) seconds, and feedback is given to the user through vibrations generated by a vibration motor 105. The vibration motor 105 is operably coupled to the processing means 102 and the data sensors 104.

The processing means 102 is further configured to trigger a notification to the user with one or more goals to be obtained, for example to move a predetermined number of steps if the user's sitting time measured by the data sensors 104 exceed a predefined threshold. The notification is triggered to ensure that the user does not continuously remain in sitting posture. The processing means 102 is also enabled to continuously analyse the real time values of the plurality of parameters, the time for which the user is in sitting posture, and number of steps taken by the user, and to suggest a plurality of personalized wellness advisories to the user. Such wellness advisories, , and are suggested or prescribed based on data specific to the user and behavioural patterns of the user. It is envisaged that the system can prescribe exercises that are customized and personalized depending on the measurements made.

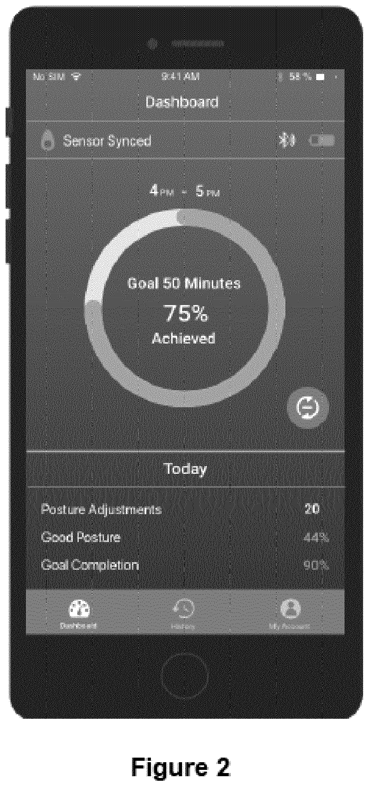

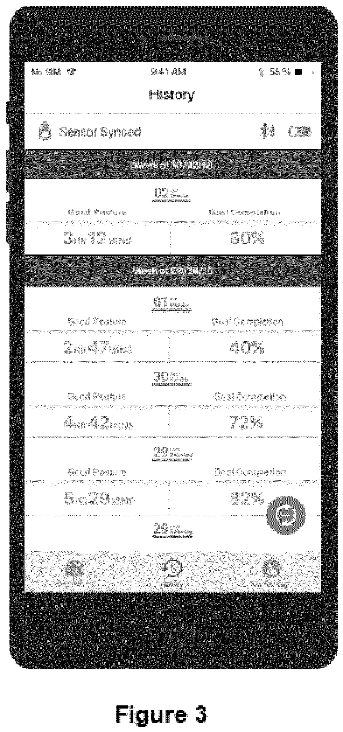

The memory means 103 has also stored on it the historic values of the plurality of parameters, the historic values of time for which the user is in sitting posture, and the historic values of number of steps moved by the user. The processing means 102 is configured to derive statistical insights from said historic values which are in turn displayed to the user through the display means 101, as illustrated in Figure 2 and Figure 3.

In an embodiment of the present invention, the aggregated and anonymized historic data of a category of users are used to train artificial intelligence and machine learning models, and the trained models are used for cluster analysis and time series analysis to predict risk mitigation strategies for the relevant category of users. Such analysis models can also be used by employers to improve employee wellness and ergonomics at workplaces.

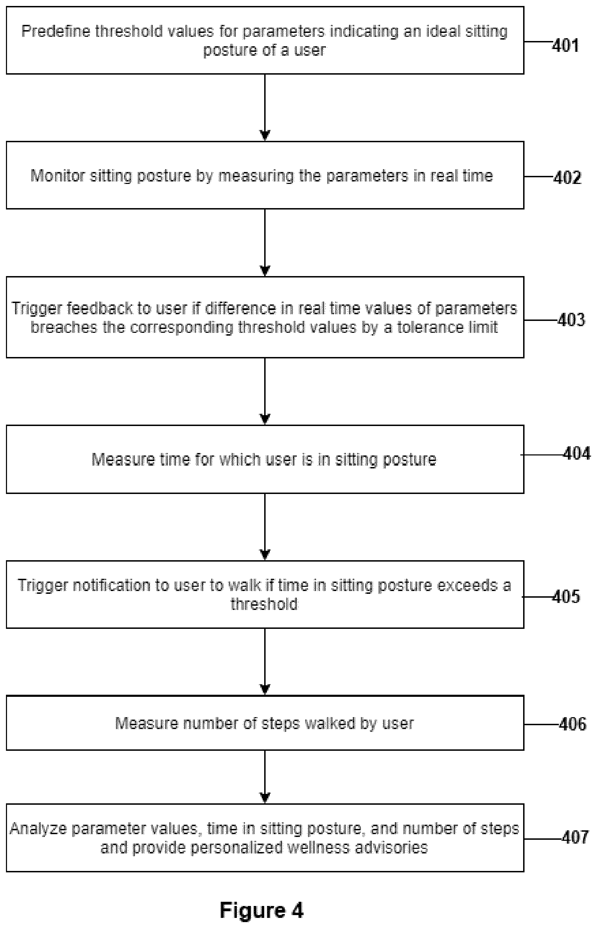

Figure 4 illustrates a method as per a preferred embodiment of the present invention. The method comprises the step of firstly predefining threshold values for a plurality of parameters corresponding to an ideal sitting posture of a user 401. The threshold values are defined based on optimal postural angles while the user is seated. The sitting posture of the user is continuously monitored by measuring the real time values of the plurality of parameters using a plurality of data sensors 402. The plurality of parameters includes for example, pitch, roll, and yaw, and the plurality of data sensors include for example, an accelerometer, a pressure sensor, a gyro-meter, and a magnetometer.

A feedback is triggered to the user if the difference in real time values of the parameters and their corresponding threshold values breaches a predefined tolerance limit 403. Such feedback is enabled after a predetermined time delay and indicates to the user that they are slouching and that their sitting posture is poor. The feedback, for example, could be in the form of vibrations generated by a vibration motor.

Further, the time for which the user is in sitting posture is measured 404, and a notification is triggered to the user with one or more goals to be obtained, for example to take a predetermined number of steps if said measured time exceeds a predefined threshold 405, and the number of steps taken by the user is then measured 406. This ensures that the user does not remain seated beyond a specific time duration. Based on real time values of parameters, the time for which the user remains seated and the number of steps taken by a user, a plurality of personalized wellness advisories are prescribed to the user 407, for example, exercises customized based on behavioural patterns of the user. It will be appreciated posture analysis and correction is separate to movement prompts. Movement away from a work station and steps targets are based only on number of minutes sitting, not the quality of sitting posture. The number of minutes 'allowed' to sit can be preset by the user.

Although the present invention has been described with reference to specific embodiments, this description is not meant to be construed in a limiting sense. Various modifications of the disclosed embodiments, as well as alternate embodiments of the subject matter, will become apparent to persons skilled in the art upon reference to the description of the subject matter. It is therefore contemplated that such modifications can be made without departing from the spirit or scope of the present invention as defined.

Further, a person ordinarily skilled in the art will appreciate that the various illustrative method steps described in connection with the embodiments disclosed herein may be implemented using electronic hardware, or a combination of hardware and software. To clearly illustrate this interchangeability of hardware and a combination of hardware and software, various illustrations and steps have been described above, generally in terms of their functionality. Whether such functionality is implemented as hardware or a combination of hardware and software depends upon the design choice of a person ordinarily skilled in the art. Such skilled artisans may implement the described functionality in varying ways for each particular application, but such obvious design choices should not be interpreted as causing a departure from the scope of the present invention.

The method described in the present disclosure may be implemented using various means. For example, the system described in the present disclosure may be implemented in hardware, firmware, software, or any combination thereof. For a hardware implementation, the processing units, or processors(s) or controller(s) may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electronic devices, other electronic units designed to perform the functions described herein, or a combination thereof.

For a firmware and/or software implementation, software code may be stored in the memory means and executed by a processor. The memory means may be implemented within the processor unit or external to the processor unit. As used herein the term "memory" refers to any type of volatile memory or non-volatile memory.

The embodiments in the invention described with reference to the drawings comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. a memory stick or hard disk. The carrier may be an electrical or optical signal which may be transmitted via an electrical or an optical cable or by radio or other means.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A system for monitoring body posture and movement, the system comprising:
a display means operatively coupled to a processing means, the display means adapted to enable a user to predefine threshold values for a plurality of parameters indicating an ideal sitting posture of the user;
a plurality of data sensors operatively coupled to the processing means, the plurality of data sensors adapted to continuously monitor the sitting posture of the user by measuring the plurality of parameters in real time, continuously measure the time for which the user is in sitting posture, and measure the number of steps taken by the user while in motion;
a memory means operatively coupled to the processing means and the display means, the memory means having stored thereon a plurality of instructions which configures the processing means to: trigger a feedback to the user after a predetermined time interval if the difference in real time values of the plurality of parameters and the predefined threshold values breaches a predefined tolerance limit, and to trigger a notification to the user to move a predetermined number of steps if the time measured by the plurality of data sensors exceed a predefined threshold, and continuously analyse the real time values of the plurality of parameters, the time for which the user is in sitting posture, and number of steps taken by the user and prescribe a plurality of personalized wellness advisories to the user.

2. The system as claimed in claim 1, further comprising a vibration motor operatively coupled to the plurality of data sensors and the processing means.

3. The system as claimed in claim 1, wherein the plurality of parameters comprises pitch, roll, and yaw.

4. The system as claimed in claim 1, wherein the processing means is further configured to derive statistical data insights from the historic values of the plurality of parameters, the historic values of time for which the user is in sitting posture, and/or the historic values of number of steps taken by the user.

5. The system as claimed in claim 1, wherein the plurality of data sensors comprises an accelerometer, a pressure sensor, a gyro-meter, and a magnetometer.

6. The system as claimed in claim 1, wherein the predetermined time period for triggering feedback to the user is selected between five and 20 seconds.

7. The system as claimed in claim 1, wherein the plurality of data sensors is positioned under the collar bone of the user.

8. The system as claimed in claim 1, wherein the threshold values for the plurality of parameters are predefined by the user based on the axial position and orientation of the plurality of data sensors.

9. A method for monitoring body posture and movement, the method comprising the steps of:
a) predefining threshold values for a plurality of parameters indicating an ideal sitting posture of a user;
b) continuously monitoring the sitting posture of the user by measuring the plurality of parameters in real time using a plurality of data sensors;
c) triggering a feedback to the user after a predetermined time interval if difference in values of the plurality of parameters measured in step (b) and the threshold values predefined in step (a), breaches a predefined tolerance limit;
d) continuously measuring the time for which the user is in sitting posture;
e) triggering a notification to the user to take a predetermined number of steps if the time measured in step (d) exceeds a predefined threshold;
f) measuring the number of steps taken by the user; and
g) continuously analysing the parameters measured in step (b), the time measured in step (d), and the number of steps measured in step (g) and prescribing a plurality of personalized wellness advisories to the user.

10. The method as claimed in claim 9, wherein the plurality of parameters comprises pitch, roll, and yaw.

11. The method as claimed in claim 9 or 10, wherein the plurality of data sensors comprises an accelerometer, a pressure sensor, a gyro-meter, and a magnetometer.

12. The method as claimed in claim 9, 10 or 11, wherein the predetermined time interval for triggering feedback to the user is selected between five and twenty seconds.
